# EUROPEAN PATENT SPECIFICATION

(11) **EP 4 150 631 B1**
(45) Date of publication and mention of the grant of the patent: **25.09.2024**
(21) Application number: 21720512.9
(22) Date of filing: 25.04.2021
(51) Int. Cl.: G16H 30/20, G16H 30/40, G16H 40/20, A61B 5/055, G01R 33/56, G01R 33/54, A61B 5/00, G01R 33/565, G16H 50/20

(54) **QUALITY CONTROL IN MEDICAL IMAGING**
QUALITÄTSKONTROLLE IN DER MEDIZINISCHEN BILDGEBUNG
CONTRÔLE QUALITÉ EN IMAGERIE MÉDICALE

(30) Priority: 12.05.2020 EP 20174016
(43) Date of publication of application: 22.03.2023
(73) Proprietor: Koninklijke Philips N.V., 5656 AG Eindhoven (NL)
(72) Inventor: HUIJBERS, Willem, 5656 AE Eindhoven (NL); MATTERS, Marco, 5656 AE Eindhoven (NL); LAMERICHS, Rudolf, Mathias, Johannes, Nicolaas, 5656 AE Eindhoven (NL); VAN EE, Raymond, 5656 AE Eindhoven (NL); DENISSEN, Adrianus, Johannes, Maria, 5656 AE Eindhoven (NL); WESTERINK, Joanne, Henriette, Desiree, Monique, 5656 AE Eindhoven (NL); LEUFKENS, Timmy, Robertus, Maria, 5656 AE Eindhoven (NL); VERSLUIS, Maarten, Jan, 5656 AE Eindhoven (NL)
(74) Representative: Philips Intellectual Property & Standards
(86) International application number: PCT/EP2021/060766
(87) International publication number: WO 2021/228533

(56) References cited:
- WO-A1-2017/102860
- US-A1- 2010 086 189
- US-A1- 2014 072 192
- US-A1- 2016 259 898
- US-A1- 2019 066 297
- US-B2- 9 836 840

## Description

### FIELD OF THE INVENTION

This invention relates to automated quality assessment and quality control of medical images.

### BACKGROUND OF THE INVENTION

It is obviously desirable to ensure the highest quality medical images are obtained, in order to improve the likelihood of making a correct diagnosis from those images. For this purpose, quality assessment and quality control measures need to be taken.

Traditionally, this is manual process, whereby the MRI system operator decides if a captured image has sufficient quality or if a re-scan is needed based on visual inspection of the images. However, the quality control steps are difficult to standardize or automate. For example, quality control of Magnetic Resonance Imaging (MRI) has proven difficult to standardize because there are no simple objective quality control metrics that capture image quality across various MRI sequences

Furthermore, quality control is not simply an objective property of the image, but also depends on the clinical goals of the physician or radiologist. Ideally quality control of MRI should be performed during acquisition, when the clinical goals are clear, but the diagnosis is still undetermined.

There is a need for a system which provides at least some automation and therefore consistency of image quality control.

WO 2017/102860 dislcoses a scanning monitoring apparatus for medical imaging, the scanning monitoring apparatus comprising a controller unit and a display, wherein the controller unit during a scanning session is configured to obtain tracking data of a subject in a medical scanner, obtain scanner data indicative of operating parameters of the medical scanner; determine an output of a verification function based on the tracking data and the scanner data; and control the scanning monitoring apparatus according to the output of the verification function.

US 2016/259898 discloses an apparatus for providing reliability for Computer Aided Diagnosis (CAD), including: a raw data collector configured to collect raw data containing an image acquired by a probe; an image reliability determiner configured to determine a reliability level of the image using the collected raw data; and a reliability provider configured to provide a user with the determined reliability of the image.

US 9836840 discloses a method for assessing the quality of medical images of at least a portion of a patient's anatomy. One method includes receiving one or more images of at least a portion of the patient's anatomy; determining one or more image properties of the received images; performing anatomic localization or modeling of at least a portion of the patient's anatomy based on the received images; obtaining an identification of one or more image characteristics associated with an anatomic feature of the patient's anatomy based on the anatomic localization or modeling; and calculating an image quality score based on the one or more image properties and the one or more image characteristics.

### SUMMARY OF THE INVENTION

The invention is defined by the claims.

According to examples in accordance with an aspect of the invention, there is provided a system for quality control in medical imaging, the system comprising:
a processor configured to:
evaluate noise sources which can adversely affect the image quality, the noise sources comprising at least two of:
   a device noise source induced by a medical imaging device during the acquisition of a medical image of a subject,
   an operator noise source induced by an operator during the acquisition of the medical image; and
   a subject noise source induced by the subject during the acquisition of the medical image; and
determine and output a quality control indicator to the operator based on the evaluated noise sources.

The "operator" is the person who operates the medical imaging equipment. This is typically different to the person who provides a medical interpretation of the images, i.e. the radiologist.

This system is able to generate a quality control indicator in real time during imaging to provide assistance to an operator or to enable automated evaluation of image quality. Furthermore, the cause of image artefacts can be determined so that corrective measures may be taken to improve image quality. The three different categories of noise source may each induce multiple possible causes of noise. Thus, a "noise source" should be understood as having a number of possible noise contributing factors. Each noise source is evaluated to identify one or more possible causes of noise which may result in artefacts in the acquired image.

Similarly, the "quality control indicator" may comprise multiple units of information, e.g. multiple quality measures.

This invention thus provides a real-time multimodal quality control system using metrics that are easy to interpret in-context and provide insight into the causes of poor signal or MRI image quality.

Preferably, the processor is configured to evaluate noise sources comprising all three of the categories listed.

The system may comprise a first arrangement for determining parameters which contribute to the device noise source, comprising one or more of:
means for determining the homogeneity of an imaging field;
a monitoring circuit for monitoring the resonance frequency;
means for detecting correct functioning of imaging transmitters and/or receivers;
an imaging device operating temperature sensor; and
a circuit for determining the energy consumption of the imaging device.

These are some of the possible ways of determining factors which contribute to the noise introduced by the device. Some of these parameters may be obtained by dedicated sensors, and others may for example be obtained based on analysis of the medical image.

The resonance frequency defines the frequency at which the atoms/molecules within the subject resonate based on an applied imaging field. The resonance frequency can change based on, for example, the temperature of the subject. For example, in MRI imaging, proton resonance frequency can be used to measure the temperature of the subject.

The system may comprise a second arrangement for determining parameters which contribute to the operator noise source, comprising one or more of:
a system for collecting data relating to the positioning, field-of-view, slice orientation, timing, and number of scans as set by the operator;
a timer for recording a time during which the operator has been working; and
an indication of a level of qualification or expertise of the operator.

These are some of the possible ways of determining factors which contribute to the noise introduced by the operator actions.

The slice orientation defines the orientation of an imaging plane with respect to the subject. For example, in MR imaging of the subject's head, threre are three basic orthogonal slice orientations: the transversal, sagittal and coronal orientiation.

The system may comprise a third arrangement for determining parameters which contribute to the subject noise source, comprising one or more of:
a sensor for sensing bodily and/or eye movements of the subject;
a respiration sensor for sensing respiration of the subject;
a skin conductance sensor;
a body temperature sensor;
a heart rate sensor for sensing the heart rate of the subject; and
an indication of the duration of the imaging process.

These are some of the possible ways of determining factors which contribute to the noise introduced by the subject actions or characteristics.

The processor is for example further configured to concurrently control a display device to present a medical image to the operator with the quality control indicator.

The quality control indicator may be a general indicator of the image quality, but more preferably it provides additional context to a user who is interpreting the medical image, for example by flagging image artefacts and indicating the likely noise source that has caused the image artefact. Recommendations may then be provided for obtaining a better quality image.

The processor may be further configured to:
determine image quality metrics for the medical image;
identify image artefacts in the medical image; and
identify the image artefacts to the operator.

The image quality metrics (such as signal to noise ratio, contrast to noise ratio etc.) relate to a general measure of image quality, and the image artefacts relate to specific image problems. These may be associated with the different noise sources. Both of these measures may be taken into account when determining the quality control indicator.

The processor may be configured to identify image artefacts using a machine learning algorithm. The machine learning algorithm is for example further configured to output a modified medical image from which the image artefacts have been removed. The machine learning may be based on deep-learning or neural networks.

In this way the system may provide improvement of image quality as well as identification of sources of noise and/or of image artefacts.

The processor may be further configured to determine the most likely source of an image quality issue based on the evaluated noise sources, and present the most likely source to the operator. The operator can then take remedial action and for example decide if a re scan will resolve the issue.

The processor may also highlight an identified image-artefact on the scan to advise the operator where on the image it can be seen. Some operators may for example not see or know where to look for artefacts.

The processor may be further configured to determine whether the medical image should be re-acquired based on the quality control indicator and present a recommendation of whether to re-acquire the medical image to the operator. This can then assist the operator in deciding whether a re-scan is appropriate.

This re-scanning can also be implemented semi-autonomously, i.e. without consent of the operator but without requiring any operator action, or if even fully autonomously, with only notification to the operator.

The processor may be further configured to:
track operator decisions during and after the acquisition of a medical image;
detect uncommon operator decisions from the operator decisions; and
request the operator for a decision rationale for the uncommon operator decisions.

These uncommon operator decisions may be indicative of operator error. The system may thus track-and-trace the medical decision further, and for example it may involve the radiologist as a further agent that can make decisions. For example, sometimes a radiologist will ask the operator for a re-scan based on the quality of the image (or a follow-up scan based on diagnostic information that requires further scanning). The system may therefore implement a decision support system, especially in relation to quality control.

The processor may thus be further configured to:
send the medical image to a radiologist for a decision referral; and
track radiologist decisions.

The system may for example use the radiologist decisions to improve the image analysis.

The processor may be further configured to create a decision map based on the operator decisions, the decision rationale and the radiologist decisions. This decision map enables the effectiveness of procedures to be evaluated.

The processor may for example control a display to show historic imaging data and/or demographic imaging data to the operator. If there are differences between the medical image and the corresponding historic imaging data and/or demographic imaging data, these differences may be displayed to the operator.

The processor may be further configured to generate a quality control report indicating how the medical device, operator and subject influence the quality of the medical imaging process. This may be used to enable local quality control over time and allow comparisons between different devices, operators and patient groups.

The invention also provides a medical imaging system, comprising:
a medical imaging device, for example an MRI scanner; and
a system for quality control as defined above, wherein the processor of the system for quality control is further configured to perform analysis of an image captured by the medical image device and determine and output the quality control indicator to the operator further based on the image analysis.

The invention also provides a computer implemented method of providing quality control in medical imaging, the method comprising:
evaluating noise sources which can adversely affect the image quality, the noise sources comprising at least two of:
   a device noise source induced by a medical imaging device during the acquisition of a medical image of a subject,
   an operator noise source induced by an operator during the acquisition of the medical image; and
   a subject noise source induced by the subject during the acquisition of the medical image; and
determining and outputting a quality control indicator to the operator based on the evaluated noise sources.

The invention also provides a computer program comprising computer program code means which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a system in accordance with an example of the invention; and
Fig. 2 shows a method in accordance with the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides system which provides quality control in medical imaging. Noise sources are evaluated which can adversely affect the image quality. The noise sources which are evaluated comprise at least two of a device noise source, an operator noise source and a subject (the patient being imaged) noise source, all creating sources of noise during the acquisition of the medical image. A quality control indicator is determined and output to the operator based on the evaluated noise sources.

To address the issue of a lack of objective quality control inherent in medical imaging, the invention is based on the concept of estimating sources of noise that are known to reduce the image quality, for example the quality of MRI images. Context can then be provided to the user as to the artefacts that are most likely, or the likely cause of a low quality image.

For the example of MR imaging, noise can originate from the MRI device, the operator or the patient who is being scanned.

With regard to the MRI device, variations in MRI device stability can cause low quality control via magnetic field inhomogeneity, radio frequency noise and malfunction of one of the RF transmitters/receivers. With regard to the operator, an MRI operator can cause low quality control due to incomplete positioning of the field-of-view. The patient can cause low quality control by in-scanner movement or other physiological fluctuations (e.g. breathing, heart rate). These patient-specific sources of noise are often larger in psychiatric patients, patients with tremors, patients with back pain, children and older adults, resulting in more noisy images and typically lower standards for quality control.

The invention is based on the recognition that an interpretation of quality, to provide a quality control indicator (i.e. a set of one or more quality control metrics) is easier when these sources of noise are assessed. The invention thus integrates an automatic estimation of noise from the MRI device, operator and patients, with quality control estimated from the MRI image itself.

When implemented on an MRI console, the estimation of noise and the quality control metrics can be evaluated in context of the clinical goals. Integration of context-sensitive quality control can then also be standardized across scanners, adopted in clinical protocols or a workflow. When successfully implemented in a workflow with a human operator, the quality control metrics can also be developed to help train machine learning algorithms to automatically detect artefacts during MRI acquisition.

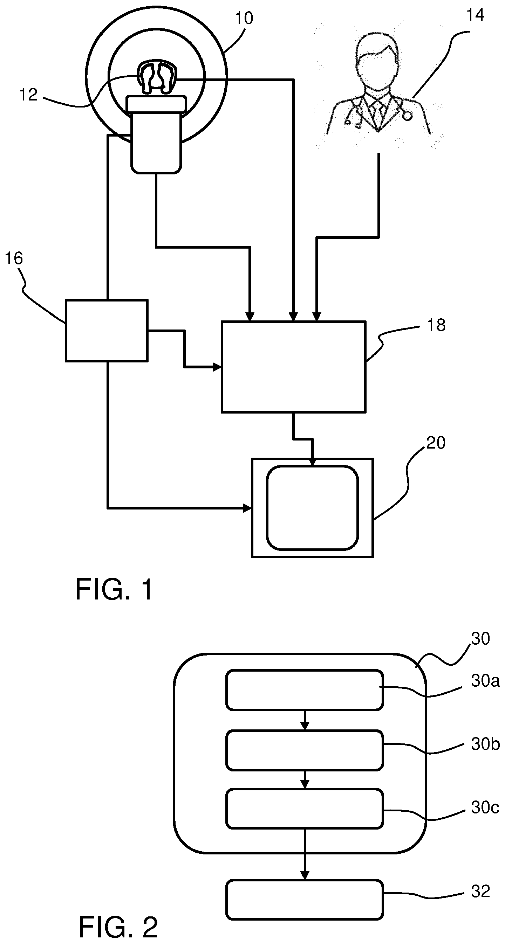

Fig. 1 shows a system in accordance with an example of the invention.

Fig. 1 shows a system for quality control in medical imaging, for use with a medical image device 10, which in this example is an MRI scanner. A subject 12 is shown in the scanner bore and the operator 14 of the device is also shown.

An imaging unit 16 generates the medical images, in conventional manner.

A processor 18 configured to evaluate noise sources which can adversely affect the image quality.

The noise sources comprise:
(i) a device noise source induced by the medical imaging device 10 during the acquisition of a medical image of a subject;
(ii) an operator noise source induced by the operator 14 during the acquisition of the medical image; and
(iii) a subject noise source induced by the subject 12 during the acquisition of the medical image.

The system may evaluate any two of these noise sources and preferably all three.

To assess these noise sources, the processor receives various sensor inputs as discussed below as well as the medical image from the imaging unit 16.

The processor then determines a quality control indicator from these noise sources. The quality control indicator is used to adapt the image to be displayed on a display 20 and/or it is used to provide an additional separate output information. This provides additional context to the operator.

This system is able to generate a quality control indicator in real time during imaging to provide assistance to the operator or to enable automated evaluation of image quality. The cause of image artefacts can be determined and can be displayed so that corrective measures may be taken to improve image quality. The three different categories of noise source may each introduce multiple possible causes of noise. Thus, each noise source is evaluated to identify one or more possible causes of noise which may result in artefacts in the acquired image.

In general terms, a first arrangement is used for determining parameters which contribute to the device noise source, such as the homogeneity of an imaging field, the resonance frequency, the correct functioning of imaging transmitters and/or receivers, the imaging device operating temperature and the energy consumption of the imaging device.

A second arrangement is used for determining parameters which contribute to the operator noise source, such as the positioning, field-of-view, slice orientation, timing, and number of scans as set by the operator, the time during which the operator has been working, and the level of qualification or expertise of the operator.

A third arrangement is used for determining parameters which contribute to the subject noise source, such as bodily and/or eye movements of the subject, respiration, perspiration, body temperature, and heart rate of the subject. Other physiological information in respect of the subject may be monitored as well as the duration of the imaging process.

The parameters may be monitored by dedicated sensors, or based on analysis of the medical image, or based on existing outputs from the imaging device.

There are for example internal MRI sensors, e.g. log-files, and these can be used to measure MRI device-induced noise. Real-time access to the MRI console for example enables access to the scan-planner set by the operator, such as data relating to the positioning, field-of-view, timing, and re-scans, in order to detect errors by the operator.

MRI image reconstruction enables a range of quality control related metrics to be gathered, including the magnetic field homogeneity, foreground/background energy, relative signal of each RF coil, sudden-spikes in the RF signal, realignment in case of a time-series and signal-to-noise estimated per MRI sequence.

The invention provides real-time analysis (and potentially presentation) of quality control metrics based on MRI reconstruction and additionally integrating noise estimates.

External sensors may also be used, such as the peripheral pulse unit for psychological measurements (respiration, heart-rate, skin-conductance) and motion, and an in-bore camera.

For the three noise sources, some specific examples will be given below of particular conditions which are monitored, how they may be monitored and what the likely impact on the image quality might be.

### 1. Device Noise Sources

### 1.1 Field inhomogeneity due to poor (active) shimming or mis-set shim current.

Detection: Automated analysis during image reconstruction of the field inhomogeneity, e.g. based on geometric distortions. Alternatively, the fat signal intensity may be monitored in MRI scans with fat suppression.

Image artefact: Image inhomogeneity at the image border, and incomplete suppression of the fat signal when fat suppression is used.

### 1.2 Heating/cooling of gradient coils

Detection: Automated analysis during scanning of the resonance frequency.

Image artefact: Signal drift and fluctuation in the time series. Geometric distortions may be observed (e.g. sheering, scaling and shifting related to eddy current artifacts).

### 1.3 Compromised RF shielding due to other peripheral devices

Detection: Automated analysis during reconstruction of RF noise, for example using spike detection on the image data in the Fourier domain, either during acquisition or combined with values from the MRI preparation phase, including the shim. .

Image artefact: Signal spikes, noisy image, poor fore/background ratio and the Zipper artefact.

### 1.4 Dropout or connection loss of MRI with peripheral devices

Detection: The software application may be used to check device connectivity, measure transit delays, and log system offsets of different devices. Solutions exist for gating/triggered MRI, when MRI acquisition is synchronized to the cardiac or respiratory cycles. In these scans, the acquisition is typically stopped temporality or entirely with connectivity loss. This might lead to longer acquisition times. The reason(s) for increases in scan time can be reported to the operator.

Image artefact: In triggered and gated MRI, there are increases in acquisition time. When peripheral devices are not linked to MRI, this can lead to timing or phase offsets in the data.

### 1.5 Active distant array coils outside the Field of View

Detection: Detection of coil movement relative the scanner, in particular the anterior body coil, by including a sensor on the coil. Automated analysis during reconstruction of image data may also be used relative to the SENSE reference scan.

Image artefact: Signal drop off, Annefact cusp artifact, star artifacts.

### 1.6 Reconstruction (run-time) error

Detection: The software application which provides automated analysis during reconstruction of data error artifacts.

Image artefact: Data Error Artifacts, Arcing or crisscross / herringbone patterns.

### 2. Operator Noise Sources

### 2.1 Placement of RF coils

Detection: A sensor in the RF coil for detecting positioning relative to the patient, combined with automated analysis during image reconstruction of the MRI scout view image.

Image artefact: Signal loss or no signal relative to target (for example the liver).

### 2.2 Placement/Size of Field of View

Detection: Automated analysis during image reconstruction of the auto-align and smart survey.

Image artefact: Phase wrap-around artifacts or clipping.

### 2.3 Placement and orientation of a saturation slab

Detection: Automated analysis during image reconstruction of saturation slab relative to the field of view and image target.

Image artefact: Poor suppression of selected spectrum.

### 2.4 Placement of slice orientation

Detection: Automated analysis during image reconstruction of fold-over or phase shift artifacts relative to slice placement.

Image artefact: Fold-over or phase shift artifacts from tissues outside the field of view propagating into the image.

### 2.5 Signal dropout due to metal object (hairpin, ring, dental, etc.)

Detection: Automated analysis during image reconstruction to detect signal dropout artefacts.

Image artefact: Local signal dropout artefacts.

### 2.6 Compromised RF shielding due to open door of the shielded room with the MRI magnet

Detection: Sensor on the door to detect open/closed state, together with automated analysis during image reconstruction of image noise, signal ratios and Zipper artifacts.

Image artefact: Noisy image, poor fore/background ratio and the Zipper artefact.

### 2.7 Mis-timing of contrast agent and MRI scan

Detection: Registration of the timing of gadolinium contrast bolus-injection, automated analysis during image reconstruction to track bolus signal using very fast low- resolution preparatory MRI scan, onset of high-resolution MRI acquisition and software for automated detection of the Maki artifact.

Image artefact: Maki artifact (a lack of signal in the center of blood vessels as compared to the edges).

### 2.8 Incorrect follow-up or diagnostic scan

Detection: Use of a dictionary to check for uncommon/unlikely combinations of scans (there may then be a suggestion generated to contact the radiologist).

Image artefact: Not visible on the image (unless it violates the operators expectation).

### 3. Patient Noise Sources

### 3.1 Voluntary patient motion.

Detection: Camera system, pressure sensors in mattress, head coil, head-phone, surface coils or other objects in contact with the patient. Analysis of the raw signal from RF coils, and automated analysis during image reconstruction of k-space trajectory and navigator scans.

Image artefact: Blurring, ghosting from moving structures, signal loss due to spin dephasing.

### 3.2 Breathing motion

Detection: Respiratory belt, pressure sensors or camera.

Image artefact: Blurring, ghosting and signal loss due to spin dephasing.

### 3.3 Breathing-dependent blood-oxygenation levels

Detection: Respiratory belt, peripheral pulse unit (PPU), breathing mask (end-tidal oxygen or carbon dioxide).

Image artefact: Confounded estimates of T2* blood-oxygenation level dependent imaging (=fMRI).

### 3.4 Eye-movements and eye blinks

Detection: Eye-tracking camera and automated analysis during image reconstruction of phase shift ghosting artifacts. For example, depending on the phase encoding direction, these artifacts can occur outside the brain, and reduce the fore/background signal ratio.

Image artefact: Phase shifts artefacts depending on the reduced fore/background signal.

### 3.5 Blood flow and blood pressure changes

Detection: Heart-rate monitor (volume clamp, PPU), inflatable cuffs and arterial applanation tonometry.

Image artefact: Changes in cerebral blood flow bias MRI acquisition (T2*, perfusion, fMRI).

### 3.6 Heart motion and heart- rate variability

Detection: Heart-rate monitor (e.g. ECG, EKG, ultrasound), PPU, and MRI flow measurement of the heart.

Image artefact: Fluctuations in an echo-planar imaging time-series.

### 3.7 Patient motion associated with the sleep/awake state of the patient or the level of anesthesia

Detection: EEG, PPG, skin-conductance or other contact electrodes, anesthetic device or via automated analysis of fMRI time-series. When combined with estimates of voluntarily motion, could also indicate insufficient levels of anesthesia.

Image artefact: Confounded estimates of cognition-related fMRI signal.

The invention is based on the use of one or more such monitoring options from at least two of the categories, thereby taking account of the interaction between at least two noise sources associated with the patient, operator and medical imaging device, and thereby enabling the causes of a reduction in image quality to be identified, as well as enabling an image quality indicator to be derived.

The image quality indicator may be integrated in a user-friendly dashboard and presented to the operator together with the acquired MRI image. In addition, the quality control metrics may be exported (e.g. within a DICOM header image) for post-hoc analysis of data quality.

The information provided (quality control indicator) may be a general indicator of the image quality, but more preferably it provides additional context to a user who is interpreting the medical image, for example by flagging image artefacts and indicating the likely noise source that has caused the image artefact. Recommendations may then be provided for obtaining a better quality image.

The real time quality control assessment from the multiple sources supports detection and interpretation of MRI artefacts, which is currently done by human visual inspection. The implementation of this real-time quality control framework can thereby greatly facilitate the development of automated artefact detection and quality control using machine learning.

In a most basic example, the system can provide context to the operator during visual inspection of MRI images, by providing the quality control indicator, for example together with estimates of noise.

In a more advanced case, the system can flag image artefacts, and identify the most likely source of noise and provide recommendation for a re-scan.

Once larger amounts of quality data are available on a single site, the system can detect outliers. These could be used for predictive maintenance and improvements in respect of a local site (i.e. a particular MRI device).

When aggregated quality data becomes available across multiple sites, scanners and (clinical) populations, the quality control metrics can be compared on distributions and local systems and operations can be developed further.

When user-feedback is safely collected at the MRI console (or in post-processing), data from image reconstruction and the quality control metrics can be used to develop and optimize machine-learning algorithms for artefact detection. When such machine learning tools are implemented in image reconstruction, for example to automatically remove image artefacts induced by motion, context-sensitive quality control metrics may be used to provide indications on "how much" and "why" data is being interpolated. These context-sensitive quality measures could then also help further improve machine learning- based image reconstruction.

User feedback can also be used to validate a machine learning algorithm at different sites, so it can generalize across different patients groups, scanners and hospitals. The invention may thus be used to test (i.e. validate) a solution.

The operator rather than the radiologist is typically the first human to evaluate scans and make autonomous on-site decisions. The operator can decide to perform re-scans based on image quality, perform immediate follow-up scans based on visible pathology, or in case of doubt or liability contact the radiologist and refer the decision.

These on-site decisions have medical consequences and costs, as approximately 5-10% of patients require additional scans during new visits, after inspection by the radiologist, while an unknown number of scans are made unnecessarily. Most decisions by the operator are made informally, logged only partially and difficult to evaluate post-hoc, unless the radiologist requests additional scans.

To address this issue, the system may be further used as a medical imaging decision assistant to track, trace and support autonomous and human decisions during the medical imaging.

In near real-time, the system can thus show scans and image analytics to the operator as explained above, additionally with a recommendation relating to the need for a re-scan or the need to contact a radiologist.

The operator makes an autonomous workflow decision to end the exam, perform a re-scan, acquire follow-up scans, or contact the radiologist. These workflow decisions can then be recorded by the system and in the case of deviations from an expected workflow, the system can request from the operator a rationale for a particular decision. This for example may apply if the system detects an uncommon operator decision.

In case of a decision of referral to the radiologist, the system may then forward information via a push application to the radiologist remotely. The remote decision by the radiologist can then also be recorded by the system.

In addition to providing a support function, the system thus operates as a medical imaging flight recorder that tracks the human decisions during the medical imaging process. The system data can then be regularly analyzed to evaluate the effectiveness of the procedures.

The system may build a periodic report (weekly, monthly, annually) that aggregates information in order to provide insight into how the medical device, operator and patients influence the quality of the medical imaging process. These aggregated reports could enable local quality control over time and allow comparisons between different devices, operators and patient groups. These reports could help identify problems or bottlenecks in the medical imaging process in a local site. In aggregated form, these reports could also allow for comparisons between devices, hospitals and regions. The reports could help prioritize development of solutions to improve the quality of medical imaging.

The system may for example determine autonomously whether the medical image should be re-acquired based on the quality control indicator and present a recommendation of whether to re-acquire the medical image to the operator. This can the assist the operator in deciding whether a re-scan is appropriate.

The system may then create a decision map based on the operator decisions, the decision rationale and the radiologist decisions. This decision map enables the effectiveness of procedures to be evaluated.

The processor may for example control a display to show historic imaging data and/or demographic imaging data to the operator. If there are differences between the medical image and the corresponding historic imaging data and/or demographic imaging data, these differences may be displayed to the operator.

The system may integrate information from a Picture Archiving and Communication System (PACS), and thereby highlight differences with previously acquired medical images to support workflow decisions. The system may also be used for providing individualize training of medical imaging technicians. The system may also be extended to assist in decisions made during image-guided radiation therapy, HIFU or other image-guided interventions.

Fig. 2 shows a computer implemented method of providing quality control in medical imaging, the method comprising:
in step 30, evaluating noise sources which can adversely affect the image quality. This comprises at least two of, and preferably all three of, the sub steps of:
in step 30a, evaluating a device noise source induced by a medical imaging device during the acquisition of a medical image of a subject,
in step 30b evaluating an operator noise source induced by an operator during the acquisition of the medical image; and
in step 30c evaluating a subject noise source induced by the subject during the acquisition of the medical image.

In step 32, a quality control indicator is determined and output to the operator based on the evaluated noise sources.

The invention has been described in connection with MRI systems. However, the invention may be applied more generally to medical imaging.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system for quality control during acquisition of a medical image, the system comprising:
a processor (18) configured to:
evaluate noise sources which can adversely affect the quality of the medical image, the noise sources comprising at least two of:
a device noise source induced by a medical imaging device (10) during the acquisition of the medical image of a subject (12);
an operator noise source induced by an operator (14) during the acquisition of the medical image; and
a subject noise source induced by the subject (12) during the acquisition of the medical image;
determine and output a quality control indicator to the operator based on the evaluated noise sources; and **characterized in that** the processor is further configured to:
identify one or more possible causes of noise which may result in artefacts in the medical image based on the evaluated noise sources.

2. The system according to claim 1, comprising a first arrangement for determining parameters which contribute to the device noise source, comprising one or more of:
means for determining the homogeneity of an imaging field when the medical image is acquired using a magnetic resonance device;
a monitoring circuit for monitoring a resonance frequency when the medical image is acquired using a magnetic resonance device;
means for detecting correct functioning of imaging transmitters and/or receivers;
an imaging device operating temperature sensor; and
a circuit for determining the energy consumption of the imaging device.

3. The system according to claim 1 or 2, comprising a second arrangement for determining parameters which contribute to the operator noise source, comprising one or more of:
a system for collecting data relating to the positioning, field-of-view, slice orientation, timing, and number of scans as set by the operator;
a timer for recording a time during which the operator has been working; and
an indication of a level of qualification or expertise of the operator.

4. The system according to any one of claims 1 to 3, comprising a third arrangement for determining parameters which contribute to the subject noise source, comprising one or more of:
a sensor for sensing bodily and/or eye movements of the subject;
a respiration sensor for sensing respiration of the subject;
a skin conductance sensor;
a body temperature sensor;
a heart rate sensor for sensing the heart rate of the subject; and
an indication of the duration of the imaging process.

5. The system according to any one of claims 1 to 4, wherein the processor is further configured to concurrently control a display device to present a medical image to the operator with the quality control indicator.

6. The system according to any one of claims 1 to 5, wherein the processor is further configured to:
determine image quality metrics for the medical image, wherein determining the quality control indicator is further based on the image quality metrics;
identify image artefacts in the medical image, wherein determining the quality control indicator is further based on the image artefacts; and
identify the image artefacts to the operator.

7. The system according to claim 6, the processor is configured to identify image artefacts using a machine learning algorithm.

8. The system according to any one of claims 1 to 7, wherein the processor is further configured to determine the most likely source of an image quality issue based on the evaluated noise sources, and present the most likely source to the operator.

9. The system according to any one of claims 1 to 8, wherein the processor is further configured to determine whether the medical image should be re-acquired based on the quality control indicator and present a recommendation of whether to re-acquire the medical image to the operator.

10. The system according to any one of claims 1 to 9, wherein the processor is further configured to:
track operator decisions during and after the acquisition of a medical image;
detect uncommon operator decisions from the operator decisions; and
request the operator for a decision rationale for the uncommon operator decisions.

11. The system according to claim 10, wherein the processor is further configured to:
send the medical image to a radiologist for a decision referral; and
track radiologist decisions.

12. The system according to any one of claims 1 to 11, wherein the processor is further configured to generate a quality control report indicating how the medical device, operator and subject influence the quality of the acquisition of the medical image, wherein generating the quality control report is based on the quality control indicator.

13. A medical imaging system, comprising:
a medical imaging device (10), for example an MRI scanner; and
a system for quality control according to any one of claims 1 to 12, wherein the processor of the system for quality control is further configured to perform analysis of an image captured by the medical image device and determine and output the quality control indicator to the operator further based on the image analysis.

14. A computer-implemented method of providing quality control during acquisition of a medical image, the method comprising:
(30) evaluating noise sources which can adversely affect the quality of a medical image, the noise sources comprising at least two of:
a device noise source (30a) induced by a medical imaging device during the acquisition of a medical image of a subject,
an operator noise source (30b) induced by an operator during the acquisition of the medical image; and
a subject noise source (30c) induced by the subject during the acquisition of the medical image;
(32) determining and outputting a quality control indicator to the operator based on the evaluated noise sources; and
identifying one or more possible causes of noise which may result in artefacts in a medical image based on the evaluated noise sources.

15. A computer program comprising computer program code means which is adapted, when said program is run on a computer, to implement the method of claim 14.

## Patentansprüche

1. System zur Qualitätskontrolle während Erhebung eines medizinischen Bildes, wobei das System umfasst:
einen Prozessor (18), der für Folgendes konfiguriert ist:
Bewerten von Rauschquellen, die die Qualität des medizinischen Bildes negativ beeinflussen können, wobei die Rauschquellen mindestens zwei der folgenden umfassen:
eine Vorrichtungsrauschquelle, die von einer medizinischen Bildgebungsvorrichtung (10) während der Erhebung des medizinischen Bildes eines Subjekts (12) induziert wird;
eine Bedienerrauschquelle, die von einem Bediener (14) während der Erhebung des medizinischen Bildes induziert wird; und
eine Subjektrauschquelle, die von dem Subjekt (12) während der Erhebung des medizinischen Bildes induziert wird;
Bestimmen und Ausgeben eines Qualitätskontrollindikators an den Bediener basierend auf den ausgewerteten Rauschquellen; und **dadurch gekennzeichnet, dass** der Prozessor weiter konfiguriert ist, um:
basierend auf den ausgewerteten Rauschquellen eine oder mehrere mögliche Rauschursachen, die zu Artefakten in dem medizinischen Bild führen können, zu identifizieren.

2. System nach Anspruch 1, das eine erste Anordnung zum Bestimmen von Parametern umfasst, die zu der Vorrichtungsrauschquelle beitragen, die eines oder mehrere der Folgenden umfasst:
Mittel zum Bestimmen der Homogenität eines Bildgebungsfelds, wenn das medizinische Bild unter Verwendung einer Magnetresonanzvorrichtung erhoben wird;
eine Überwachungsschaltung zum Überwachen einer Resonanzfrequenz, wenn das medizinische Bild unter Verwendung einer Magnetresonanzvorrichtung erhoben wird;
Mittel zum Erheben eines korrekten Funktionierens von Bildgebungssendern und/oder -empfängern;
einen Bildgebungsvorrichtungssensor; und
eine Schaltung zum Bestimmen des Energieverbrauchs der Bildgebungsvorrichtung.

3. System nach Anspruch 1 oder 2, das eine zweite Anordnung zum Bestimmen von Parametern umfasst, die zu der Bedienerrauschquelle beitragen, die eines oder mehrere der Folgenden umfasst:
ein System zum Sammeln von Daten in Bezug auf Positionierung, Sichtfeld, Schichtausrichtung, Zeitpunkt und Anzahl der Abtastungen, wie vom Bediener eingestellt;
einen Timer zum Aufzeichnen einer Zeit, während der der Bediener gearbeitet hat; und
eine Angabe eines Qualifikations- oder Fachkompetenzniveaus des Bedieners.

4. System nach einem der Ansprüche 1 bis 3, das eine dritte Anordnung zum Bestimmen von Parametern umfasst, die zu der Subjektrauschquelle beitragen, die eines oder mehrere der Folgenden umfasst:
einen Sensor zum Erfassen von Körper- und/oder Augenbewegungen des Subjekts;
einen Atmungssensor zum Erfassen der Atmung des Subjekts;
einen Hautleitfähigkeitssensor;
einen Temperatursensor;
einen Herzfrequenzsensor zum Erfassen der Herzfrequenz des Subjekts; und
eine Angabe der Dauer des Bildgebungsprozesses.

5. System nach einem der Ansprüche 1 bis 4, wobei der Prozessor weiter dazu konfiguriert ist, gleichzeitig eine Anzeigevorrichtung zu steuern, um dem Bediener ein medizinisches Bild mit dem Qualitätskontrollindikator zu präsentieren.

6. System nach einem der Ansprüche 1 bis 5, wobei der Prozessor weiter dazu konfiguriert ist:
Bildqualitätsmetriken für das medizinische Bild zu bestimmen, wobei Bestimmen des Qualitätskontrollindikators weiter auf den Bildqualitätsmetriken basiert;
Bildartefakte in dem medizinischen Bild zu identifizieren, wobei Bestimmen des Qualitätskontrollindikators weiter auf den Bildartefakten basiert; und
die Bildartefakte für den Bediener zu identifizieren.

7. System nach Anspruch 6, wobei der Prozessor dazu konfiguriert ist, Bildartefakte unter Verwendung eines maschinellen Lernalgorithmus zu identifizieren.

8. System nach einem der Ansprüche 1 bis 7, wobei der Prozessor weiter dazu konfiguriert ist, die wahrscheinlichste Quelle eines Bildqualitätsproblems basierend auf den ausgewerteten Rauschquellen zu bestimmen und dem Bediener die wahrscheinlichste Quelle zu präsentieren.

9. System nach einem der Ansprüche 1 bis 8, wobei der Prozessor weiter dazu konfiguriert ist, basierend auf dem Qualitätskontrollindikator zu bestimmen, ob das medizinische Bild erneut erhoben werden sollte, und dem Bediener eine Empfehlung zu präsentieren, ob das medizinische Bild erneut erhoben werden sollte.

10. System nach einem der Ansprüche 1 bis 9, wobei der Prozessor weiter dazu konfiguriert ist:
Entscheidungen des Bedieners während und nach der Erhebung eines medizinischen Bildes zu verfolgen;
ungewöhnliche Bedienerentscheidungen aus den Bedienerentscheidungen zu erfassen; und
von dem Bediener eine Entscheidungsbegründung für die ungewöhnlichen Bedienerentscheidungen anzufordern.

11. System nach Anspruch 10, wobei der Prozessor weiter dazu konfiguriert ist:
das medizinische Bild an einen Radiologen zum Treffen einer Entscheidung zu senden; und
die Entscheidungen des Radiologen zu verfolgen.

12. System nach einem der Ansprüche 1 bis 11, wobei der Prozessor weiter dazu konfiguriert ist, einen Qualitätskontrollbericht zu erzeugen, der angibt, wie die medizinische Vorrichtung, der Bediener und das Subjekt die Qualität der Erhebung des medizinischen Bildes beeinflussen, wobei Erzeugen des Qualitätskontrollberichts auf dem Qualitätskontrollindikator basiert.

13. Medizinisches Bildgebungssystem, umfassend:
eine medizinische Bildgebungsvorrichtung (10), beispielsweise einen MRI-Scanner; und
ein System zur Qualitätskontrolle nach einem der Ansprüche 1 bis 12, wobei der Prozessor des Systems zur Qualitätskontrolle weiter dazu konfiguriert ist, eine Analyse eines von der medizinischen Bildgebungsvorrichtung aufgenommenen Bildes durchzuführen und den Qualitätskontrollindikator weiter basierend auf der Bildanalyse zu bestimmen und an den Bediener auszugeben.

14. Computergestütztes Verfahren zum Bereitstellen einer Qualitätskontrolle während Erhebung eines medizinischen Bildes, wobei das Verfahren Folgendes umfasst:
(30) Auswerten von Rauschquellen, die die Qualität eines medizinischen Bildes negativ beeinflussen können, wobei die Rauschquellen mindestens zwei der folgenden umfassen:
eine Vorrichtungsrauschquelle (30a), die von einer medizinischen Bildgebungsvorrichtung während der Erhebung eines medizinischen Bildes eines Subjekts induziert wird,
eine Bedienerrauschquelle (30b), die von einem Bediener während der Erhebung des medizinischen Bildes induziert wird; und
eine Subjektrauschquelle (30c), die von dem Subjekt während der Erhebung des medizinischen Bildes induziert wird;
(32) Bestimmen und Ausgeben eines Qualitätskontrollindikators an den Bediener basierend auf den bewerteten Rauschquellen; und
Identifizieren einer oder mehrerer möglicher Rauschursachen, die zu Artefakten in einem medizinischen Bild führen können, basierend auf den ausgewerteten Rauschquellen.

15. Computerprogramm, umfassend Computerprogrammcodemittel, die angepasst sind, wenn das Programm auf einem Computer läuft, das Verfahren nach Anspruch 14 zu implementieren.

## Revendications

1. Système de contrôle de qualité lors de l'acquisition d'une image médicale, le système comprenant :
un processeur (18) configuré pour :
évaluer des sources de bruit qui peuvent nuire à la qualité de l'image médicale, les sources de bruit comprenant au moins deux parmi :
une source de bruit de dispositif induite par un dispositif d'imagerie médicale (10) lors de l'acquisition de l'image médicale d'un sujet (12) ;
une source de bruit d'opérateur induite par un opérateur (14) lors de l'acquisition de l'image médicale ; et
une source de bruit de sujet induite par le sujet (12) lors de l'acquisition de l'image médicale ;
déterminer et transmettre un indicateur de contrôle de qualité à l'opérateur sur la base des sources de bruit évaluées ; et **caractérisé en ce que** le processeur est en outre configuré pour :
identifier une ou plusieurs causes possibles de bruit qui peuvent entraîner des artéfacts dans l'image médicale sur la base des sources de bruit évaluées.

2. Système selon la revendication 1, comprenant un premier agencement pour déterminer des paramètres qui contribuent à la source de bruit de dispositif, comprenant un ou plusieurs des éléments suivants :
des moyens pour déterminer l'homogénéité d'un champ d'imagerie lorsque l'image médicale est acquise à l'aide d'un dispositif à résonance magnétique ;
un circuit de surveillance pour surveiller une fréquence de résonance lorsque l'image médicale est acquise à l'aide d'un dispositif de résonance magnétique ;
des moyens pour détecter le fonctionnement correct d'émetteurs et/ou de récepteurs d'imagerie ;
un capteur de température de fonctionnement de dispositif d'imagerie ; et
un circuit pour déterminer la consommation d'énergie du dispositif d'imagerie.

3. Système selon la revendication 1 ou 2, comprenant un deuxième agencement pour déterminer des paramètres qui contribuent à la source de bruit d'opérateur, comprenant un ou plusieurs des éléments suivants :
un système de collecte de données relatives à un positionnement, un champ de vision, une orientation de tranches, une synchronisation et un nombre de balayages définis par l'opérateur ;
une minuterie pour enregistrer un temps, pendant lequel l'opérateur a travaillé ; et
une indication d'un niveau de qualification ou d'expertise de l'opérateur.

4. Système selon l'une quelconque des revendications 1 à 3, comprenant un troisième agencement pour déterminer des paramètres qui contribuent à la source de bruit de sujet, comprenant un ou plusieurs des éléments suivants :
un capteur pour détecter des mouvements corporels et/ou oculaires du sujet ;
un capteur respiratoire pour détecter la respiration du sujet ;
un capteur de conductance cutanée ;
un capteur de température corporelle ;
un capteur de fréquence cardiaque pour détecter la fréquence cardiaque du sujet ; et
une indication de la durée du processus d'imagerie.

5. Système selon l'une quelconque des revendications 1 à 4, dans lequel le processeur est en outre configuré pour commander simultanément un dispositif d'affichage afin de présenter une image médicale à l'opérateur avec l'indicateur de contrôle de qualité.

6. Système selon l'une quelconque des revendications 1 à 5, dans lequel le processeur est en outre configuré pour :
déterminer des mesures de qualité d'image pour l'image médicale, dans lequel la détermination de l'indicateur de contrôle de qualité est en outre basée sur les mesures de qualité d'image ;
identifier des artéfacts d'image dans l'image médicale, dans lequel la détermination de l'indicateur de contrôle de qualité est en outre basée sur les artéfacts d'image ; et
identifier les artéfacts d'image pour l'opérateur.

7. Système selon la revendication 6, le processeur est configuré pour identifier des artéfacts d'image à l'aide d'un algorithme d'apprentissage automatique.

8. Système selon l'une quelconque des revendications 1 à 7, dans lequel le processeur est en outre configuré pour déterminer la source la plus probable d'un problème de qualité d'image sur la base des sources de bruit évaluées, et présenter la source la plus probable à l'opérateur.

9. Système selon l'une quelconque des revendications 1 à 8, dans lequel le processeur est en outre configuré pour déterminer si l'image médicale doit être ré-acquise sur la base de l'indicateur de contrôle de qualité et présenter une recommandation quant à la nécessité de ré-acquérir l'image médicale pour l'opérateur.

10. Système selon l'une quelconque des revendications 1 à 9, dans lequel le processeur est en outre configuré pour :
suivre les décisions d'opérateur pendant et après l'acquisition d'une image médicale ;
détecter des décisions d'opérateur inhabituelles à partir des décisions d'opérateur ; et
demander à l'opérateur une justification de décisions des décision d'opérateur inhabituelles.

11. Système selon la revendication 10, dans lequel le processeur est en outre configuré pour :
envoyer l'image médicale à un radiologue pour prise de décision ; et
suivre les décisions de radiologue.

12. Système selon l'une quelconque des revendications 1 à 11, dans lequel le processeur est en outre configuré pour générer un rapport de contrôle de qualité indiquant comment le dispositif médical, l'opérateur et le sujet influencent la qualité de l'acquisition de l'image médicale, dans lequel la génération du rapport de contrôle de qualité est basée sur l'indicateur de contrôle de qualité.

13. Système d'imagerie médicale, comprenant :
un dispositif d'imagerie médicale (10), par exemple un scanner IRM ; et
un système de contrôle de qualité selon l'une quelconque des revendications 1 à 12, dans lequel le processeur du système de contrôle de qualité est en outre configuré pour effectuer une analyse d'une image capturée par le dispositif d'imagerie médicale et déterminer et transmettre l'indicateur de contrôle de qualité à l'opérateur en se basant en outre sur l'analyse d'images.

14. Procédé mis en oeuvre par ordinateur pour fournir un contrôle de qualité lors de l'acquisition d'une image médicale, le procédé comprenant :
(30) l'évaluation de sources de bruit qui peuvent nuire à la qualité d'une image médicale, les sources de bruit comprenant au moins deux des éléments suivants :
une source de bruit de dispositif (30a) induite par un dispositif d'imagerie médicale lors de l'acquisition d'une image médicale d'un sujet,
une source de bruit d'opérateur (30b) induite par un opérateur lors de l'acquisition de l'image médicale ; et
une source de bruit de sujet (30c) induite par le sujet lors de l'acquisition de l'image médicale ;
(32) la détermination et la transmission d'un indicateur de contrôle de qualité à l'opérateur sur la base des sources de bruit évaluées ; et
l'identification d'une ou plusieurs causes possibles de bruit qui peuvent entraîner des artéfacts dans une image médicale sur la base des sources de bruit évaluées.

15. Programme informatique comprenant un moyen de code de programme informatique qui est conçu, lorsque ledit programme est exécuté sur un ordinateur, pour mettre en oeuvre le procédé de la revendication 14.
